# EUROPEAN PATENT APPLICATION

(11) **EP 4 559 423 A1**
(43) Date of publication of application: **28.05.2025**
(21) Application number: 23841823.0
(22) Date of filing: 13.04.2023
(51) Int. Cl.: A61B 18/20, A61N 5/06

(54) **LASER HAIR REMOVAL HANDLE**

(30) Priority: 18.07.2022 CN 202221851850 U
(71) Applicant: Focuslight Technologies Inc., Xi'an, Shaanxi 710077 (CN)
(72) Inventor: LYU, Yiping, Xi'an, Shaanxi 710077 (CN); ZHAO, Yiduo, Xi'an, Shaanxi 710077 (CN); WEI, Yao, Xi'an, Shaanxi 710077 (CN); YANG, Kai, Xi'an, Shaanxi 710077 (CN); CAO, Heng, Xi'an, Shaanxi 710077 (CN)
(74) Representative: Basfeld, Rainer
(86) International application number: PCT/CN2023/087958
(87) International publication number: WO 2024/016750

(57) **Abstract**

The present application provides a laser hair removal handle, relating to the technical field of laser hair removal equipment, comprising: a water-access block, a hollow metal structural member, and a fast-axis collimating mirror; by installing a laser on the transverse side of the L-shaped water-access block, and installing a hollow metal structural member on the vertical side of the L-shaped water-access block, diffuse reflected light formed by the laser light irradiating the skin enters into a cavity of the hollow metal structural member, and because the hollow metal structural member is a metal material As the hollow metal structural member is a metal material, it effectively absorbs the energy of the diffusely reflected light and reduces the effect of the diffusely reflected light on the emitted laser light, and the laser light emitted from the laser is collimated by using a fast-axis collimating mirror, which results in lower energy loss compared to the optical waveguide of the prior art.

## Description

### Cross-Reference to Related Applications

The present application claims the priority of the Chinese patent application filed with the Chinese Patent Office on July 18, 2020, with the application number CN202221851850.9, titled as " Laser Hair Removal Handle", the entire contents of which is incorporated herein by reference.

### Technical Field

The present application relates to the technical field of laser hair removal equipment, and more particular to a laser hair removal handle.

### Background Art

Semiconductor laser hair removal technology can safely, quickly and permanently remove unwanted body hair. Semiconductor laser hair removal is based on the theory of selective photothermal effect, so that a specific wavelength of laser light penetrates the epidermis, without damaging the epidermal hair follicles, the melanin in the hair shaft selectively absorbs the laser energy, the hair follicles are heated to coagulation and necrosis, and the hairs will no longer grow.

However, the use of optical waveguides in laser hair removal handles causes the energy of the laser that reaches the window piece, to undergo a loss of energy of about 10 to 15 percent.

### Summary

The purpose of this application is to provide a laser hair removal handle to alleviate the problem that the use of optical waveguides in laser hair removal handles in the prior art will causes an energy loss of about 10% to 15% of the laser energy reaching the window piece.

In a first aspect, the present application provides a laser hair removal handle comprising: a water pass-through block, a hollow metal structural member, and a fast-axis collimating mirror;
Said the water-passing block has an L-shaped cross-sectional shape, with the transverse side of the L-shaped water-passing block being configured for mounting the laser;
Said the hollow metal structure passes through the vertical side of the L-shaped water-passing block, and the hollow metal structure is closely connected to the water-passing block;
Said the fast axis collimator is arranged on the light output side of said laser, and is configured to collimate the laser light emitted by said laser, and the collimated laser light passes through the cavity of the hollow metal structure and irradiates the skin, and the hollow metal structure is configured to absorb the light energy diffusely reflected by the skin.

In optional implementations,
Said the water-passing block has a penetration hole configured to install the hollow metal structure block, and the penetration hole is located on the vertical side of the L-shaped water-passing block.

In optional implementations,
Said laser hair removal handle further comprises a refrigeration head;
said refrigeration head is located on the vertical side of said L-shaped water-passing block away from said laser and said refrigeration head is connected to said water-passing block;
Said refrigeration head is sleeved on the hollow metal structural member, and there is a gap between the refrigeration head and the hollow metal structural member.

In optional implementations,
Said laser hair removal handle further comprises a window piece;
Said window piece is located on the side of said refrigeration head away from said water-passing block and said window piece is connected to said refrigeration head, and said window piece is provided with a high permeability membrane on the side of said window piece near said water-passing block.

In optional implementations,
Said laser hair removal handle further comprises a semiconductor cooler;
Said semiconductor cooler is sleeved on the hollow metal structural member, and the semiconductor cooler is located between the vertical edge of the L-shaped water-passing block and the refrigeration head.

In optional implementations,
The cross-sectional shape of said semiconductor cooler is set in the form of a rectangular ring.

In optional implementations,
Said laser hair removal handle further comprises a semiconductor cooler;
Said bottom of the refrigeration head has an extension edge, and the semiconductor cooler is located on the transverse edge of the extension edge and the L-shaped water-passing block.

In optional implementations,
Said hollow metal structural member is set as a rectangular cylindrical structure, and both ends of the hollow metal structural member have openings.

In optional implementations,
A sealing ring is sandwiched between said laser and the transverse edge of said L-shaped water-passing block.

In optional implementations,
One end of said laser away from said fast axis collimator mirror has a wiring terminal.

The present application provides a laser hair removal handle, comprising: a water-passing block, a hollow metal structural member, and a fast-axis collimating mirror; by installing a laser on the transverse edge of the L-shaped water-passing block, and installing a hollow metal structural member on the vertical side of the L-shaped water-passing block, the diffusely reflected light formed after the laser irradiates the skin enters into a cavity of the hollow metal structural member. Since the hollow metal structural member is a metal material, it effectively absorbs the energy of the diffusely reflected light, reducing the impact of the diffusely reflected light on the emitted laser light. Additionally, the laser light emitted from the laser is collimated using a fast-axis collimating mirror, which has a lower energy loss compared to the optical waveguide in the prior art. This alleviates the technical problem of using the optical waveguide in the laser hair removal handle in the existing technology, which cause about 10% to 15% energy loss of the laser energy reaching the window piece.

### Brief Description of Drawings

In order to illustrate the technical solutions of the embodiments of the present application more clearly, the drawings needed to be used in the embodiments will be briefly introduced below. It should be understood that the following drawings only show some embodiments of the present application, and therefore it should not be regarded as a limitation on the scope. For those skilled in the art, other related drawings can also be obtained according to these drawings without any creative efforts.
FIG. 1 shows a cross-sectional view of the overall structure of a laser hair removal handle provided by an embodiment of the present application;
FIG. 2 shows a schematic diagram of the overall structure of a laser hair removal handle provided by an embodiment of the present application;
FIG. 3 shows a schematic diagram of the structure of a laser hair removal handle provided by an embodiment of the present application in an axial side view;
FIG. 4 shows a schematic diagram of an exploded structure of a laser hair removal handle provided by an embodiment of the present application;
FIG. 5 shows a schematic diagram of the structure of a laser hair removal handle provided by an embodiment of the present application under another embodiment.

### Reference Numbers:

100-water- passing block; 200-hollow metal structural member; 300-fast-axis collimating mirror; 400-laser; 410- wiring terminal; 500-refrigeration head; 510-extension edge; 600-window piece; 700-semiconductor cooler.

### Detailed Description of Embodiments

In order to make the purposes, technical solutions and advantages of the embodiments of the present application clearer, the technical solutions in the embodiments of the present application will be clearly and completely described below with reference to the drawings in the embodiments of the present application. Obviously, the described embodiments are some, but not all, of embodiments of the present application. Generally, the components of the embodiments of the present application described and illustrated in the drawings herein may be arranged and designed in a variety of different configurations.

Thus, the following detailed description of the embodiments of the present application provided in the drawings is not intended to limit the scope of the present application as claimed, but is merely representative of selected embodiments of the present application. Based on the embodiments of the present application, all other embodiments obtained by those skilled in the art without creative efforts shall fall within the protection scope of the present application.

It should be noted that similar reference numbers and letters refer to similar items in the following drawings, and thus once an item is defined in one drawing, it is not required to further define and explain it in subsequent drawings.

In the description of this application, it is noted that the terms "center", "top", "bottom", "left", "right", "vertical", "horizontal", "inner", "outer" "right", "vertical", "horizontal", "inside", "outside", etc. indicate an orientation. "The indicated orientation or positional relationship is based on the orientation or positional relationship shown in the accompanying drawings, or the orientation or positional relationship in which the product of the application is customarily placed when used, and is only for the purpose of facilitating the description of the application and simplifying the description, and is not intended to indicate or imply that the device or element referred to must have a specific orientation, be constructed and operated with a specific orientation, and therefore is not to be construed as a limitation of the application. Therefore, it is not to be construed as a limitation of this application. Furthermore, the terms "first", "second", "third", etc. are configured to distinguish descriptions only and are not to be understood as indicating or implying relative importance.

In addition, the terms "horizontal", "vertical", "overhanging", etc., do not mean that the parts are required to be absolutely horizontal or overhanging, but may be slightly inclined. For example, "horizontal" simply means that its orientation is more horizontal than "vertical" and does not mean that the structure must be perfectly horizontal, but may be slightly inclined.

In the description of the present application, it should also be noted that the terms "set up", "installed", "connected" are used unless otherwise expressly specified and qualified, "connected" are to be understood in a broad sense, for example, as a fixed connection, a removable connection, or a connection in one piece; a mechanical connection, or an electrical connection; a direct connection, or an indirect connection through an intermediate medium; or a connection within two elements. For a person of ordinary skill in the art, the specific meaning of the above terms in the present application may be understood in specific cases.

In conjunction with the accompanying drawings, some embodiments of the present application are described in detail below. In the absence of conflict, the following embodiments and features in the embodiments can be combined with each other.

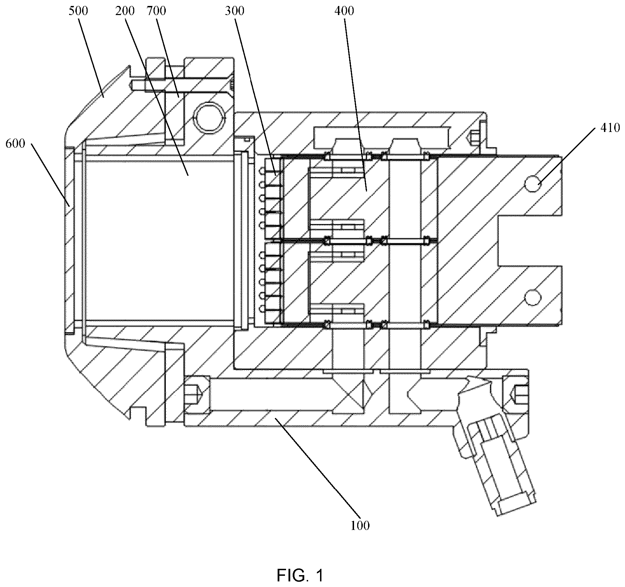

As shown in FIG1, the present embodiment provides a laser hair removal handle comprising: a water-passing block 100, a hollow metal structural member 200 and a fast axis collimator 300; the cross-sectional shape of the water-passing block 100 is L-shaped, and the transverse side of the L-shaped water-passing block 100 is configured to install a laser 400; the hollow metal structural member 200 passes through the vertical side of the L-shaped water-passing block 100, and the hollow metal structural member 200 is closely connected to the water-passing block 100; the fast axis collimator 300 is arranged on the light emitting side of the laser 400, and the fast axis collimator 300 is configured to collimate the laser emitted by the laser 400, and the collimated laser passes through the cavity of the hollow metal structural member 200 to irradiate the skin, and the hollow metal structural member 200 is configured to absorb the light energy diffusely reflected by the skin.

Specifically, the shape of the water-passing block 100 is set in an L-shape, the laser 400 is mounted on the transverse edge of the L-shaped water-passing block 100, and the bolt reaches upwardly from the bottom of the transverse edge of the L-shaped water-passing block 100 into the laser 400, so that the laser 400 can be fixed to the transverse edge of the L-shaped water-passing block 100.

The hollow metal structural member 200 is mounted on the vertical side of the L-shaped water-passing block 100, the hollow metal structural member 200 is a rectangular cylinder structure, both ends of the hollow metal structural member 200 have openings, and the hollow metal structural member 200 is a metal material that can effectively absorb the energy of the diffusely reflected light.

A fast axis collimator 300 is provided between the laser 400 and the hollow metal structure 200. The fast axis collimator 300 is integrated on the laser 400. The fast axis collimator 300 is used to replace the optical waveguide, thereby reducing the optical energy loss from the laser 400 to the window. The fast axis collimator 300 is lighter than the optical waveguide, thereby achieving lightweight laser hair removal handle.

The laser hair removal handle provided in this embodiment comprising: a water-passing block 100, a hollow metal structural member 200 and a fast axis collimator 300; by installing a laser 400 on the transverse side of the L-shaped water-passing block 100 and installing the hollow metal structural member 200 on the vertical side of the L-shaped water-passing block 100, the diffuse reflected light formed after the laser is irradiated on the skin enters the cavity of the hollow metal structural member 200, and since the hollow metal structural member 200 is made of metal material, the energy of the diffuse reflected light is effectively absorbed, reduces the impact of the diffuse reflected light on the emitted laser, and the laser emitted by the laser 400 is collimated by the fast axis collimator 300, compared with the optical waveguide in the prior art, the energy loss is lower, which alleviates the technical problem that the use of the optical waveguide in the laser hair removal handle in the prior art will cause the energy of the laser 400 reaching the window piece to suffer an energy loss of about 10% to 15%.

In optional embodiments, the water-passing block 100 has a penetration hole configured to install a hollow metal structural member, and the
penetration hole is located on the vertical edge of the L-shaped water-passing block 100.

Specifically, a penetration hole is opened on the vertical side of the L-shaped water-passing block 100, and the hollow metal structural member 200 is mounted in the penetration hole, so that the openings on both sides of the hollow metal structural member 200 face the laser 400 and the refrigeration head 500, respectively, and the laser emitted by the laser 400 passes through the fast-axis collimating mirror 300 and then passes through the hollow metal structural member 200 and irradiates onto the skin.

In optional embodiments, the laser hair removal handle further comprises a refrigeration head 500; the refrigeration head 500 is disposed on a side of the vertical edge of the L-shaped water-passing block 100 away from the laser 400 and the refrigeration head 500 is connected to the water-passing block 100; The refrigeration head 500 is installed on the hollow metal structural component 200, and the refrigeration head 500 has a gap between the refrigeration head 500 and the hollow metal structural member 200.

Specifically, the refrigeration head 500 is mounted on the vertical side of the L-shaped water-passing block 100 by bolts, and a hole is opened in the middle of the refrigeration head 500, which is hooded on the hollow metal structural member 200, and because the refrigeration head 500 has a void between the refrigeration head 500 and the hollow metal structural member 200, the heat is effectively prevented from being conducted to the refrigeration head 500.

In optional embodiments, the laser hair removal handle further comprises a window piece 600; the window piece 600 is located on a side of the refrigeration head 500 away from the water-passing block 100, and the window piece 600 is connected to the refrigeration head 500, and the window piece 600 is provided with a high permeability membrane on the side of the window piece 600 near the water-passing block 100.

Specifically, the window piece 600 is mounted in an opening in the middle of the refrigeration head 500, the window piece 600 is made of sapphire, the inner side of the window piece 600 is coated with a high-transmission film, and the therapeutic surface in contact with the skin does not need to be coated.

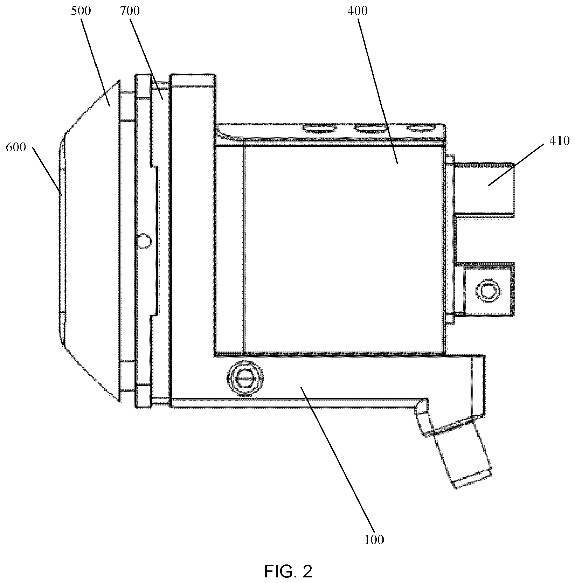

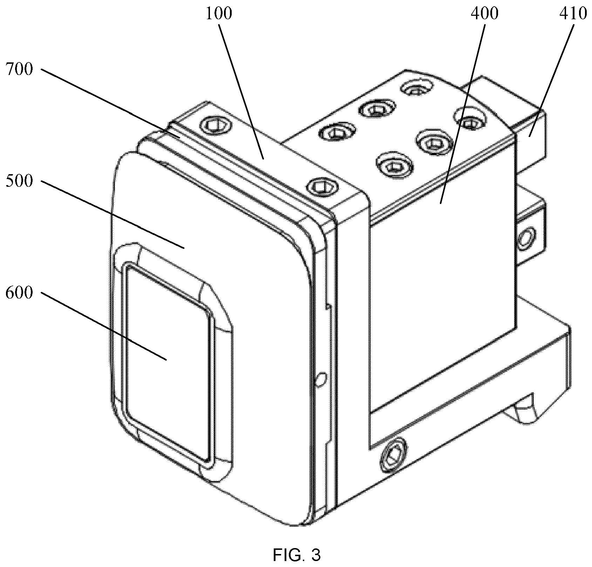

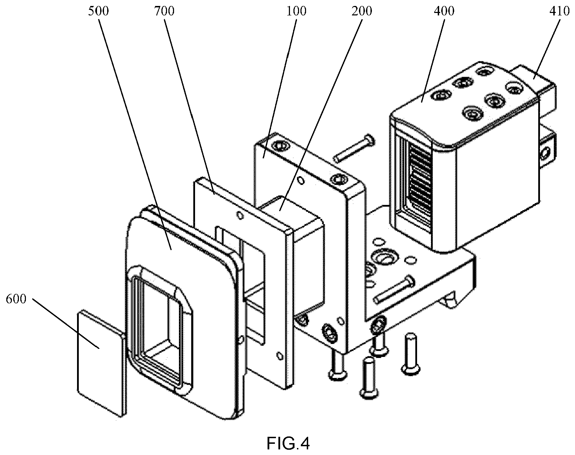

As shown in FIGS. 2, 3, and 4, in an optional embodiment, the laser hair removal handle further comprises a semiconductor cooler 700; the semiconductor cooler 700 is sleeved on the hollow metal structure 200, and the semiconductor cooler 700 is disposed between the vertical edge of the L-shaped water-passing block 100 and the refrigeration head 500; and the cross-sectional shape of the semiconductor cooler 700 is set to be rectangular ring.

Specifically, as an alternative embodiment, the semiconductor cooler 700 is set in the shape of a rectangular ring-shaped, and the rectangular ring-shaped semiconductor cooler 700 is sleeved on the hollow metal structure member 200, so that the semiconductor cooler 700 is sandwiched between the refrigeration head 500 and the vertical edges of the L-shaped water-passing block 100, and the rectangular ring-shaped semiconductor cooler 700 is set closer to the window piece 600 that is in contact with the skin, so that the thermal conduction path is shorter and the cross-sectional area is larger, enhancing the cooling capacity for the skin is stronger.

In addition, the vertical edge of the L-shaped water-passing block 100 is configured closer to the refrigeration head 500 to provide heat dissipation not only to the laser 400, but also to the rectangular ring-shaped semiconductor cooler 700, and the hollow metal structural member 200.

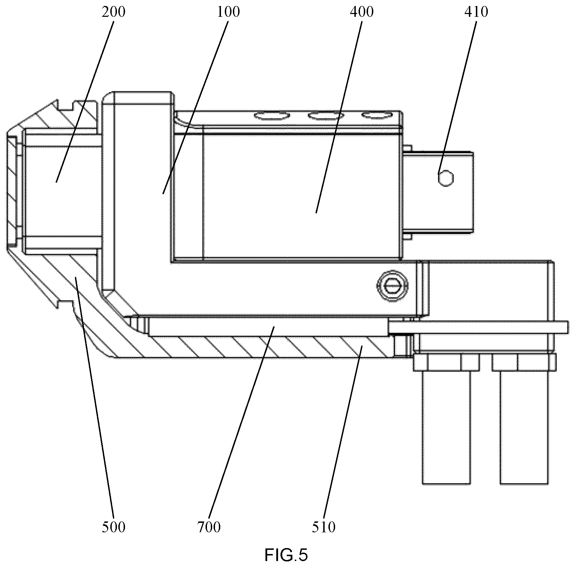

As shown in FIG. 5, in an optional embodiment, the laser hair removal handle further comprises a semiconductor cooler 700; the bottom of the refrigeration head 500 has an extension edge 510, and the semiconductor cooler 700 is disposed on a transverse edge of the extension edge 510 closer to the L-shaped water-passing block 100.

Specifically, as an alternative embodiment, the semiconductor cooler 700 is configured in a block shape, the bottom of the refrigeration head 500 extends in the direction closer to the laser 400 to form an extension edge 510, and the block-shaped semiconductor cooler 700 is mounted between the transverse edge of the L-shaped water-passing block 100 and the extension edge510, using a downward pressure type semiconductor cooler 700.

In an optional embodiment, a sealing ring is sandwiched between the laser 400 and the transverse edge of the L-shaped water-passing block 100.

Specifically, the sealing ring is installed between the transverse edge of the L-shaped water-passing block 100 and the laser 400, and the compression ratio of the sealing ring is 15% to 30% to prevent water leakage.

In an optional embodiment, the laser 400 has a wiring terminal 410 at one end away from the fast axis collimator 300.

Specifically, the laser 400 has a wiring terminal 410, which is electrically connected to the electrode and can be protected by a reverse diode.

The laser hair removal handle provided in this embodiment integrates a fast axis collimating mirror 300 on the laser 400, greatly reducing the energy loss from the output of the laser 400 to the window piece 600, and making the handle lighter and smaller; the rectangular semiconductor cooler 700 enhances the cooling effect in contact with the skin; The setting of the hollow metal structural member 200 effectively absorbs the energy of diffuse reflected light, enhances the cooling capacity of the skin in contact, and the L-shaped water-passing block 100 further improves the heat dissipation capacity.

Finally, it should be noted that the above embodiments are only used to illustrate the technical solutions of the present application, not to limit them; although the present application has been described in detail with reference to the foregoing embodiments, a person of ordinary skill in the art should understand that he or she can still make modifications to the technical solutions as recorded in the foregoing embodiments, or make equivalent substitutions for some or all of the technical features therein; and such modifications or substitutions The modifications or substitutions do not take the essence of the corresponding technical solutions out of the scope of the technical solutions of the embodiments of the present application.

### Industrial Applicability

The present application provides a laser hair removal handle, which uses hollow metal structural components as metal materials to effectively absorb the energy of diffuse reflected light, thereby reducing the impact of diffuse reflected light on the emitted laser, and use a fast axis collimating mirror to collimate the laser emitted by the laser. Compared with the optical waveguide in the prior art, the energy loss is lower, alleviating the technical problem where using optical waveguides in the laser hair removal handle in the prior art could cause about 10% to 15% energy loss of the laser energy reaching the window piece.

## Claims

1. A laser hair removal handle, **characterized in that** it comprises: a water-passing block (100), a hollow metal structural member (200) and a fast-axis collimator (300); Said the cross-sectional shape of the water-passing block (100) is L-shaped, and the transverse side of the L-shaped water-passing block (100) is configured to mount a laser (400); Said the hollow metal structural member (200) pass through the vertical side of the L-shaped water-passing block (100), and the hollow metal structural member (200) is closely connected to the water-passing block (100);
Said the fast-axis collimator (300) is arranged on the light-emitting side of the laser (400), said the fast-axis collimator (300) is configured to collimate the laser light emitted by the laser (400), and the collimated laser light passes through the cavity of said hollow metal structural member (200) and irradiates the skin, said hollow metal structural member (200) is configured to absorb the light energy diffusely reflected by the skin.

2. The laser hair removal handle according to claim 1, **characterized in that**
said water- passing block (100) has penetration holes configured to mount said hollow metal structural block, with said penetration holes being located on the vertical side of said water- passing block (100) which is L-shaped.

3. The laser hair removal handle according to claim 1 or 2, **characterized in that** said laser hair removal handle further comprises a refrigeration head (500); Said refrigeration head (500) is located on the side of the vertical side of said water-passing block (100) of L-shape away from said laser (400), and said refrigeration head (500) is connected to said water-passing block (100); Said refrigeration head (500) is sleeved on said hollow metal structural member (200) and there is a gap between said refrigeration head (500) and said hollow metal structural member (200).

4. The laser hair removal handle according to claim 3, **characterized in that**
said laser hair removal handle further comprises a window piece (600);
Said window piece (600) is located on the side of said refrigeration head (500) away from said water-passing block (100), and said window piece (600) is connected to said refrigeration head (500), and said window piece (600) is provided with a high permeability film on the side of said window piece (600) near said water-passing block (100).

5. The laser hair removal handle according to claim 4, **characterized in that**
said laser hair removal handle further comprises a semiconductor cooler (700); Said semiconductor cooler (700) is sleeved on said hollow metal structural member (200) and said semiconductor cooler (700) is located between the vertical side of said water-passing block (100) in L-shape and said refrigeration head (500).

6. The laser hair removal handle according to claim 5, **characterized in that**
the cross-sectional shape of said semiconductor cooler (700) is configred in the form of a rectangular ring.

7. The laser hair removal handle according to claim 4, **characterized in that** said laser hair removal handle further comprises a semiconductor cooler (700); The bottom of said refrigeration head (500) has an extension edge (510), and said semiconductor cooler (700) is located on the transverse edge of said extension edge (510) adjacent to said water-passing block (100) of L-shape.

8. The laser hair removal handle according to any one of claims 1 to 7, **characterized in that** said hollow metal structural member (200) is configured as a rectangular cylinder structure, and both ends of said hollow metal structural member (200) have openings.

9. The laser hair removal handle according to any one of claims 1 to 8, **characterized in that** a sealing ring is sandwiched between the laser (400) and the transverse edge of the L-shaped water-passing block (100).

10. The laser hair removal handle according to any one of claims 1 to 9, **characterized in that** one end of said laser (400) away from said fast axis collimator (300) has a wiring terminal (410).
